# EUROPÄISCHE PATENTSCHRIFT

(11) **EP 1 051 147 B1**
(45) Veröffentlichungstag und Bekanntmachung des Hinweises auf die Patenterteilung: **22.12.2004**
(21) Anmeldenummer: 99903670.0
(22) Anmeldetag: 22.01.1999
(51) Int. Cl.: A61K 7/09

(54) **VERFAHREN ZUR DAUERHAFTEN VERFORMUNG VON KERATINFASERN**
METHOD FOR PERMANENTLY DEFORMING KERATIN FIBERS
PROCEDE POUR DEFORMER DE FACON PERMANENTE DES FIBRES KERATINIQUES

(30) Priorität: 31.01.1998 DE 19803803
(43) Veröffentlichungstag der Anmeldung: 15.11.2000
(73) Patentinhaber: Cognis Deutschland GmbH & Co. KG, 40589 Düsseldorf (DE)
(72) Erfinder: BIGORRA, Joaquin, E-08203 Sabadell (ES); PRAT, Esther, E-08238 Alella (ES)
(86) Internationale Anmeldenummer: PCT/EP1999/000408
(87) Internationale Veröffentlichungsnummer: WO 1999/038477

(56) Entgegenhaltungen:
- EP-A- 0 885 607
- WO-A-97/47284
- DE-A- 19 504 502
- DE-C- 19 801 086

## Beschreibung

### Gebiet der Erfindung

Die Erfindung betrifft ein Verfahren zur dauerhaften Verformung von Keratinfasem unter Einsatz von Esterquats als Emulgatoren sowie deren Verwendung zur Herstellung von Wellmitteln.

### Stand der Technik

Die dauerhafte Verformung von Keratinfasem wird üblicherweise so durchgeführt, daß man die Faser mechanisch verformt und die Verformung durch geeignete Hilfsmittel festlegt. Vor und/oder nach dieser Verformung behandelt man die Faser mit der wäßrigen Zubereitung einer keratinreduzierenden Substanz und spült nach einer Einwirkungszeit mit Wasser oder einer wäßrigen Lösung. In einem zweiten Schritt behandelt man dann die Faser mit der wäßrigen Zubereitung eines Oxidationsmittels. Nach einer Einwirkungszeit wird auch dieses ausgespült und die Faser von den mechanischen Verformungshilfsmitteln (Wickler, Papilloten) befreit.

Die wäßrige Zubereitung des Keratinreduktionsmittels ist üblicherweise alkalisch eingestellt, damit die Faser quillt und auf diese Weise ein tiefes Eindringen der keratinreduzierenden Substanz in die Faser ermöglicht wird. Die keratinreduzierende Substanz spaltet einen Teil der Disulfid-Bindungen des Keratins zu -SH-Gruppen, so daß es zu einer Lockerung der Peptidvemetzung und infolge der Spannung der Faser durch die mechanische Verformung zu einer Neuorientierung des Keratingefüges kommt. Unter dem Einfluß des Oxidationsmittels werden erneut Disulfid-Bindungen geknüpft, und auf diese Weise wird das Keratingefüge in der vorgegebenen Verformung neu fixiert.

Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten Haaren angewendet werden.

Wenngleich dieses als Dauerwelle bezeichnete Verfahren heute in großem Umfang angewendet wird, werden dafür nach wie vor Mittel eingesetzt, die hinsichtlich einer Reihe von Punkten nicht als optimal angesehen werden können. Insbesondere ist man bestrebt, unter Beibehaltung der gewünschten Umformleistung die bei strapaziertem, insbesondere bei oxidativ vorbehandeltem, Haar auftretenden Schädigungen, die bis hin zum Haarbruch gehen können, zu verringern. Gleiches gilt für die in manchen Fällen auftretenden Probleme im Kopfhautbereich. Ein weiteres Problem besteht femer darin, daß die Zubereitungen insbesondere bei Temperaturlagerung leicht eindicken und dann nicht mehr problemlos anwendbar sind.

Die Aufgabe der vorliegenden Erfindung hat daher darin bestanden, ein Verfahren zur dauerhaften Verformung von Keratinfasern, insbesondere von menschlichem Haar, zur Verfügung zu stellen, welches frei von den oben geschilderten Nachteilen ist.

### Beschreibung der Erfindung

Gegenstand der Erfindung ist ein Verfahren zur dauerhaften Verformung von Keratinfasern, bei welchem man die Faser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült, welches sich dadurch auszeichnet, daß die wäßrige Zubereitung der keratinreduzierenden Substanz und/oder des Oxidationsmittels Tenside vom Esterquat-Typ enthält.

Es wurde nun überraschenderweise gefunden, daß eine wesentliche Verringerung der Schädigung des Haares unter Erhalt oder sogar Steigerung der Umformleistung erzielt werden kann, wenn die eingesetzten Mittel Emulgatoren vom Typ der Esterquats enthalten. Die Erfindung schließt die Erkenntnis ein, daß sich die Performance weiter verbessern läßt, wenn man die Esterquats zusammen mit nichtionischen Tensiden vom Typ der Alkyl- und/oder Alkenyloligoglykoside, der Fettsäure-N-alkylpolyhydroxyalkylamide und/oder Proteinhydrolysate einsetzt.

### Esterquats

Unter der Bezeichnung "Esterquats" werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quatemiert. Aus der Deutschen Patentschrift **DE-C1 4308794** (Henkel) ist überdies ein Verfahren zur Herstellung fester Esterquats bekannt, bei dem man die Quatemierung von Triethanolaminestern in Gegenwart von geeigneten Dispergatoren, vorzugsweise Fettalkoholen, durchführt. Übersichten zu diesem Thema sind beispielsweise von R.Puchta et al. in **Tens.Surf.Det., 30, 186 (1993),** M.Brock in **Tens.Surf.Det. 30, 394 (1993),** R.Lagerman et al. in **J.Am.Oil.Chem.Soc., 71, 97 (1994)** sowie I.Shapiro in **Cosm.Toil. 109, 77 (1994)** erschienen.

Die quatemienen Fettsäuretriethanolaminestersalze folgen der Formel (I), 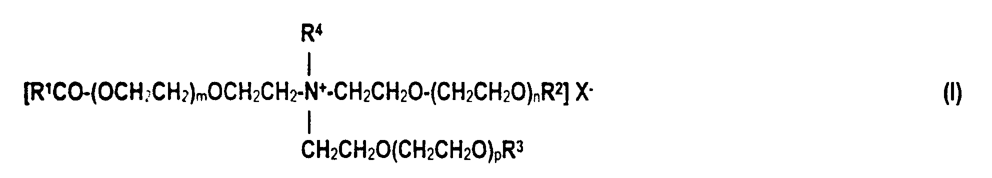 in der R¹CO fur einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe. m. n und p in Summe für 0 oder Zahlen von 1 bis 12, q für Zahlen von 1 bis 12 und X für Halogenid. Alkylsuifat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsaure, Myristinsäure, Palmitinsäure, Isostearinsäure, Stearinsäure, Ölsäure, Elaidinsäure, Arachinsaure. Behensäure und Erucasäure sowie deren technische Mischungen, wie sie beispielsweise bei der Druckspaltung natürlicher Fette und Öle anfallen. Vorzugsweise werden technische C_{12/18}-Kokosfettsauren und insbesondere teilgehärtete C_{16/18}-Talg- bzw. Palmfettsäuren sowie elaidinsäurereiche C_{16/18}-Fettsäureschnitte eingesetzt. Zur Herstellung der quatemierten Ester können die Fettsäuren und das Triethanolamin im molaren Verhältnis von 1,1 : 1 bis 3 : 1 eingesetzt werden, Im Hinblick auf die anwendungstechnischen Eigenschaften der Esterquats hat sich ein Einsatzverhältnis von 1,2 : 1 bis 2.2 . 1, vorzugsweise 1,5 : 1 bis 1,9 : 1 als besonders vorteilhaft erwiesen. Die bevorzugten Esterquats stellen technische Mischungen von Mono-, Di- und Triestem mit einem durchschnittlichen Veresterungsgrad von 1,5 bis 1,9 dar und leiten sich von technischer C_{16/18}- Talg- bzw. Palmfettsäure (lodzahl 0 bis 40) ab. Aus anwendungstechnischer Sicht haben sich quaternierte Fettsäuretriethanolaminestersalze der Formel (I) als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht.

Neben den quaternierten Fettsäuretriethanolaminestersalzen kommen als Esterquats ferner auch quatemierte Estersalze von Fettsäuren mit Diethanolalkylaminen der Formel (II) in Betracht, 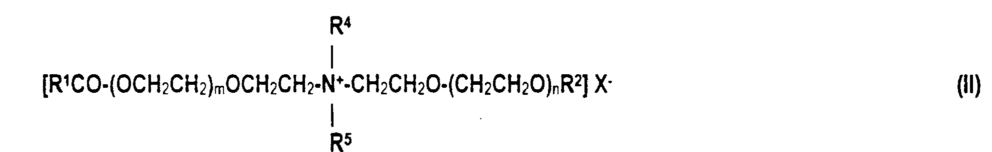 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quaternierten Estersalze von Fettsäuren mit 1,2-Dihydroxypropyldialkylaminen der Formel **(III)** zu nennen, 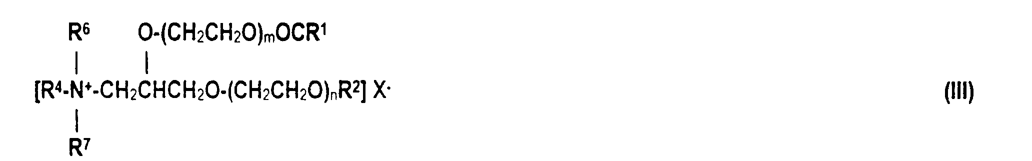 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Veresterungsgrades gelten die für (I) genannten Beispiele auch für die Esterquats der Formeln **(II)** und **(III)**. Üblicherweise gelangen die Esterquats in Form 50 bis 90 Gew.-%iger alkoholischer Lösungen in den Handel, die bei Bedarf problemlos mit Wasser verdünnt werden können. Im Sinne des erfindungsgemäßen Verfahrens können die Esterquats in Mengen von 0,1 bis 20, vorzugsweise 0,5 bis 10 und insbesondere 1 bis 5 Gew.-%jeweils bezogen auf die keratinreduzierende Substanz bzw. das Oxidationsmittel - eingesetzt werden.

### Alkyl- und/oder Alkenyloligoglykoside

Alkyl- und Alkenyloligoglykoside stellen bekannte nichtionische Tenside dar, die der Formel (IV) folgen,

**R**^{**8**}**O-[G]**_{**p**} (IV)

in der R⁸ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in **Starch/Stärke 45, 281 (1993)**, B.Salka in **Cosm.Toil. 108, 89 (1993)** sowie J.Kahre et al. in **SÖFW-Journal Heft 8, 598 (1995)** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel (IV) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁸ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R⁸ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Im Sinne des erfindungsgemäßen Verfahrens können die Alkyl- und/oder Alkenyloligoglykoside in Mengen von 0,1 bis 10, vorzugsweise 0,5 bis 5 und insbesondere 1 bis 3 Gew.-% - jeweils bezogen auf die keratinreduzierende Substanz bzw. das Oxidationsmittel - eingesetzt werden.

### Fettsäure-N-alkylpolyhydroxyalkylamide

Fettsäure-N-alkylpolyhydroxyalkylamide stellen nichtionische Tenside dar, die der Formel **(V)** folgen, 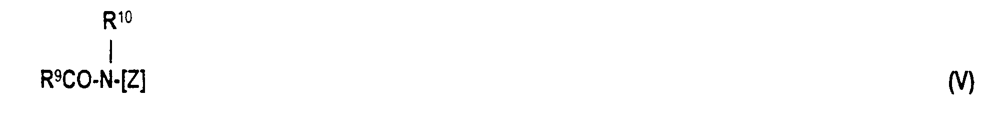 in der R⁹CO für einen aliphatischen Acylrest mit 6 bis 22 Kohlenstoffatomen, R¹⁰ für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H.Kelkenberg findet sich in **Tens.Surf.Deterg. 25, 8 (1988).**

Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(VI)** wiedergegeben werden:

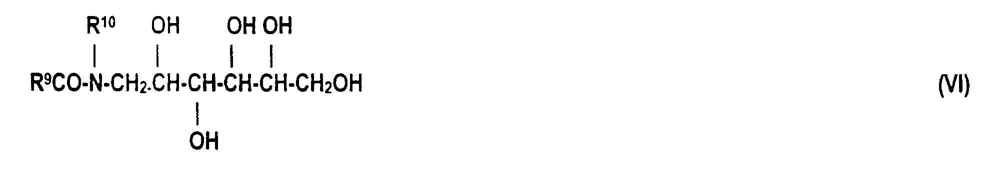

Vorzugsweise werden als Fettsäure-N-alkylpolyhydroxyalkylamide Glucamide der Formel (VI) eingesetzt, in der R⁹ für eine Alkylgruppe steht und R¹⁰CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel **(VI)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

Im Sinne des erfindungsgemäßen Verfahrens können die Fettsäure-N-alkylpolyhydroxyalkylamide in Mengen von 0,1 bis 10, vorzugsweise 0,5 bis 5 und insbesondere 1 bis 3 Gew.-% - jeweils bezogen auf die keratinreduzierende Substanz bzw. das Oxidationsmittel - eingesetzt werden.

### Proteinhydrolysate

Proteinhydrolysate stellen Abbauprodukte von tierischen oder pflanzlichen Proteinen, beispielsweise Collagen, Elastin oder Keratin und vorzugsweise Mandel- und Kartoffelprotein sowie insbesondere Weizen-, Reis- und Sojaprotein dar, die durch saure, alkalische und/oder enzymatische Hydrolyse gespalten werden und danach ein durchschnittliches Molekulargewicht im Bereich von 600 bis 4000, vorzugsweise 2000 bis 3500 aufweisen. Obschon Proteinhydrolysate in Ermangelung eines hydrophoben Restes keine Tenside im klassischen Sinne darstellen, finden sie wegen ihrer dispergierenden Eigenschaften vielfach Verwendung zur Formulierung oberflächenaktiver Mittel. Übersichten zu Herstellung und Verwendung von Proteinhydrolysaten sind beispielsweise von G.Schuster und A.Domsch in **Seifen Öle Fette Wachse 108, 177 (1982)** bzw. **Cosm.Toil. 99, 63 (1984),** von H.W. Steisslinger in **Parf.Kosm. 72, 556 (1991)** und F.Aurich et al. in **Tens.Surf.Det. 29, 389 (1992)** erschienen. Vorzugsweise werden pflanzliche Proteinhydrolysate auf Basis von Weizengluten oder Reisprotein eingesetzt, deren Herstellung in den beiden Deutschen Patentschriften **DE-C1 19502167** und **DE-C1 19502168** (Henkel) beschrieben wird. Die Proteinhydrolysate können im Sinne des erfindungsgemäßen Verfahrens auch kationisch oder anionisch modifiziert sein.

Kationische Derivate erhält man dann durch Umsetzung mit Verbindungen, die üblicherweise quartäre Ammoniumgruppen tragen oder durch Umsetzung mit entsprechenden Aminen und anschließende Quatemierung. Eine Reihe solcher quartärer Proteinhydrolysate sind als Handelsprodukte erhältlich, beispielsweise:
- kationisches Kollagenhydrolysat, beispielsweise das unter der Bezeichnung Lamequat®L auf dem Markt befindliche Produkt (INCI-Bezeichnung: Lauryldimonium Hydroxypropylamino Hydrolyzed Animal Protein; Chemische Fabrik Grünau)
- kationisches Keratinhydrolysat, beispielsweise das unter der Bezeichnung Croquat® auf dem Markt befindliche Produkt (INCI-Bezeichnung: Cocodimonium Hydroxypropyl Hydrolyzed Keratin; Croda)
- kationisches Weizenhydrolysat, erhältlich unter der Bezeichnung Hydrotriticum®QL (CTFA-Bezeichnung: Lauryldimonium Hydroxypropyl Hydrolized Wheat Protein; Croda) das unter der Bezeichnung Crotein®Q erhältliche Produkt, gemäß INCI ein "Steartrimonium Hydrolyzed Animal Protein" (Croda) sowie das als Lexein®Q X 3000 (Inolex) erhältliche quatemierte Eiweißhydrolysat.

Anionische Derivate von Proteinhydrolysaten werden üblicherweise durch Umsetzung der Proteinhydrolysate mit organischen Säuren erhalten. Solche Säuren sind beispielsweise Ölsäure, Myristinsäure, Undecylensäure, Kokosfettsäure und Abietinsäure. Die Kondensationsprodukte können auch in Form von Salzen, insbesondere Natrium-, Kalium- und Triethanolaminsalzen vorliegen. Solche Kondensationsprodukte auf Basis Kollagenhydrolysat tragen auch die INCI-Bezeichnungen Oleoyl Hydrolyzed Animal Protein, Myristoyl Hydrolyzed Animal Protein, Oleoyl Hydrolyzed Animal Collagen, Potassium Coco Hydrolyzed Animal Protein, TEA Abietoyl Hydrolyzed Animal Collagen, Potassium Undecylenoyl Hydrolyzed Animal Collagen und TEA Coco Hydrolyzed Animal Collagen. Handelsprodukte sind beispielsweise Lamepon®LPO, Lamepon®4 SK, Lamepon®UD, Lamepon®460, Lamepon®PA TR, Lamepon®ST 40 und Lamepon®S (Grünau) sowie Lexein®A 240, Lexein®S 620 und Lexein®A 520 (Inolex). Kondensationsprodukte von Elastinhydrolysaten mit Fettsäuren wie beispielsweise Laurinsäure (INCI-Bezeichnung: Lauroyl Hydrolyzed Animal Elastin) können ebenfalls eingesetzt werden. Crolastin®AS (Croda) ist ein entsprechendes Marktprodukt. Unter der Bezeichnung Promois EGCP erhältlich ist ein Potassium Cocoyl Hydrolyzed Wheat Protein; (Seiwa). Weitere erfindungsgemäß einsetzbare Handelsprodukte sind Lexein®A 200 (Inolex), Lamepon®PO-TR, Lamepon®PA-K, Lamepon®S-MV und Lamepon®S-TR (Grünau) und Crotein®CCT (Croda).

Im Sinne des erfindungsgemäßen Verfahrens können die gegebenenfalls kationisch oder anionisch modifizierten Proteinhydrolysate in Mengen von 0,1 bis 10, vorzugsweise 0,5 bis 5 und insbesondere 1 bis 3 Gew.-% - jeweils bezogen auf die keratinreduzierende Substanz bzw. das Oxidationsmittel - eingesetzt werden.

### Gewerbliche Anwendbarkeit

Unter Einsatz von Esterquats werden Well- und Fixiermittel erhalten, die nicht nur mild sind und ausgezeichnete Eigenschaften in der Haarverformung aufweisen, sondern auch bei Temperaturlagerung nicht eindicken und eine vorteilhafte Viskosität nach Brookfield im Bereich von 4.000 bis 7.000 mPas aufweisen. Ein weiterer Gegenstand der Erfindung betrifft die Verwendung von Esterquats zur Herstellung von Well- und Fixiermitteln, in denen sie in Mengen von jeweils 0,1 bis 20, vorzugsweise 0,5 bis 10 und insbesondere 1 bis 5 Gew.-% enthalten sein können.

Im weiteren werden folgende Bezeichnungen verwendet:
- "Wellmittel" für die wäßrige Zubereitungen der keratinreduzierenden Substanz.
- "Zwischenspülung" für die erste Spülung und
- "Fixiermittel" für die wäßrige Zubereitung des Oxidationsmittels.

Weiterhin werden die Einzelheiten der erfindungsgemäßen Lehre anhand von Dauerwellmitteln geschildert. Die entsprechenden Mittel eignen sich aber in gleichem Maße und mit den gleichen Vorteilen zum Glätten von natürlich gekräuselten oder gewellten Haaren.

### Wellmittel

Wellmittel, zu deren Herstellung die Esterquats verwendet werden können und die im Sinne des erfindungsgemäßen Verfahrens Anwendung finden, enthalten zwingend die als keratinreduzierende Substanzen bekannten Mercaptane. Solche Verbindungen sind beispielsweise Thioglykolsäure, Thiomilchsäure, Thioäpfelsäure, Mercaptoethansulfonsäure sowie deren Salze und Ester, Cysteamin, Cystein, Bunte Salze und Alkalisalze der schwefligen Säure. Bevorzugt geeignet sind die Alkali- oder Ammoniumsalze der Thioglykolsäure und/oder der Thiomilchsäure sowie die freien Säuren. Diese werden in den Wellmitteln bevorzugt in Konzentrationen von 0,5 bis 1,0 Mol/kg bei einem pH-Wert von 5 bis 12, insbesondere von 7 bis 8,5, eingesetzt. Die Wellmittel können als gebrauchsfertige Mischungen formuliert werden, die vom Friseur oder Endverbraucher direkt angewendet werden können. Es hat sich in manchen Fällen aber als vorteilhaft oder notwendig erwiesen, wenn die Mittel als sogenannte 2-Komponenten-Mischungen formuliert werden, die erst vom Anwender zum gebrauchsfertigen Wellmittel vermischt werden. In diesem Fall enthält eine Formulierung das Reduktionsmittel in einem geeigneten Träger, z.B. Wasser oder einer Emulsion.

### Fixiermittel

Zwingender Bestandteil der Fixiermittel, zu deren Herstellung man die Esterquats Verwenden kann und die ebenfalls Anwendung im erfindungsgemäßen Verfahren finden, sind Oxidationsmittel, z. B. Natriumbromat, Kaliumbromat, Wasserstoffperoxid, und die zur Stabilisierung wäßriger Wasserstoffperoxidzubereitungen üblichen Stabilisatoren. Der pH-Wert solcher wäßriger Wasserstoffperoxid-Zubereitungen, die üblicherweise etwa 0,5 bis 3,0 Gew.-% H₂O₂ enthalten, liegt bevorzugt bei 2 bis 4; er wird durch anorganische Säuren, bevorzugt Phosphorsäure, eingestellt. Fixiermittel auf Bromat-Basis enthalten die Bromate üblicherweise in Konzentrationen von 1 bis 10 Gew.-% eingesetzt und der pH-Wert der Lösungen wird auf 4 bis 7 eingestellt. Gleichfalls geeignet sind Fixiermittel auf enzymatischer Basis (Peroxidasen), die keine oder nur geringe Mengen an Oxidationsmitteln, insbesondere H₂O₂, enthalten. Es kann bevorzugt sein, das Oxidationsmittel als 2-Komponenten-System zu formulieren.

Die beiden Komponenten, von denen die eine bevorzugt eine Wasserstoffperoxidiösung oder eine wäßrige Lösung eines anderen Oxidationsmittels ist und die andere die übrigen Bestandteile enthält, werden ebenfalls erst kurz vor der Anwendung vermischt.

### Tenside

Sowohl Well- als auch Fixiermittel können weitere Tenside in untergeordneten Mengen enthalten. Als untergeordnete Mengen werden erfindungsgemäß Mengen von weniger als 70 %, insbesondere weniger 50 % an Aktivsubstanz verstanden. Als weitere Tenside kommen prinzipiell alle für Haarbehandlungsmittel, insbesondere auf dem Verformungsgebiet, bekannten Tenside in Betracht. Dies sind.

**Anionische Tenside.** wie beispielsweise Seifen, Alkylbenzolsulfonate, Alkansulfonate, Olefinsulfonate, Alkyletnersuifonate. Glycerinethersulfonate, α-Methylestersulfonate, Sulfofettsäuren, Alkylsulfate, Fettalkoholetnersulfate. Glycerinethersulfate, Fettsäureethersulfate, Hydroxymischethersulfate, Monoglycend(ether)sulfate. Fettsäureamid(ether)-sulfate, Mono- und Dialkyl-sulfosuccinate, Mono- und Dialkylsulfosuccinamate. Sulfotriglyceride, Amidseifen, Ethercarbonsäuren und deren Salze, Fettsaureisethionate. Fettsauresarcosinate, Fettsäuretauride, N-Acylaminosäuren, wie beispielsweise Acyllactylate. Acyltartrate. Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester. Fettsäureamidpolyglycolether, Fettaminpolyglycolether, alkoxylierte Triglyceride, Mischether bzw Mischformale, gegebenenfalls partiell oxidierte Glucoronsäurederivate, Polyolfettsäureester, Zuckerester. Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217** verwiesen.

Der Fachmann wird dabei bevorzugt solche Tenside auswählen, die aufgrund ihres geringen Reizpotentials oder ihrer Quellwirkung vorteilhaft sind. In einer besonders vorteilhaften Ausführungsform der vorliegenden Erfindung enthalten aber weder Well- noch Fixiermittel weitere Tenside außer Esterquats, Alkylpolyglykosiden, Fettsäure-N-alkylglucamiden und pflanzlichen Proteinhydrolysaten. Auch die Zwischenspülung enthält bevorzugt neben Wasser und gelösten Salzen keine weiteren Komponenten. Weiterhin kann es von Vorteil sein, wenn Wellmittel und Fixiermittel auf der gleichen Tensidbasis aufgebaut sind.

### Hilfs- und Zusatzstoffe

Weiterhin können die Wellmittel alle für diesen Zweck bekannten Inhaltsstoffe enthalten, wie z.B. Perlglanzwachse, Stabilisatoren, Konsistenzgeber, Verdickungsmittel, Polymere, Siliconverbindungen, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Parfümöle, Farbstoffe und dergleichen.

Als **Perlglanzwachse** kommen beispielsweise in Frage : Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinyl-imidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quatemierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

**Als anionische, zwitterionische, amphotere und nichtionische Polymere** kommen beispielsweise Vinylacetat/Crotonsäure-Copolymere, VinylpyrrolidonNinylacrylat-Copolymere, Vinylacetat/Butylmaleat/Isobomylacrylat-Copolymere, Methylvinylether/Maleinsäureanhydrid-Copolymere und deren Ester, unvemetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinyl-pyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen. Als **Quell- und Penetrationsmittel** für wäßrige Phasen können Montmorillonite, Clay Mineralstoffe, Pemulen, Glycerin, Propylenglykolmonoethylether, Carbonate, Hydrogencarbonate, Guanidine, Harnstoffe, primäre, sekundäre und tertiäre Phosphate sowie alkylmodifizierte Carbopoltypen (Goodrich) dienen.
Unter **UV-Lichtschutzfiftern** sind organische Substanzen zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäureisopentylester, 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion;

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C). Weitere geeignete UV-Lichtschutzfilter sind der Übersicht von P.Finkel in **SÖFW-Journal 122, 543 (1996)** zu entnehmen.

Zur Verbesserung des Fließverhaltens können ferner **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäur. Als **Strukturanten** eignen sich beispielsweise Glucose oder Maleinsäure. Als **Komplexbildner** können EDTA, NTA und Phosphonsäuren eingesetzt werden. Als **Trübungsmittel** dient beispielsweise Latex, als **Treibmittel** können u.a. Propan-Butan-Gemische, Distickstoffoxid, Dimethylether oder Luft eingesetzt werden.

Als **Parfümöle** seien genannt die Extrakte von Blüten (Lavendel, Rosen, Jasmin, Neroli), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Moschus, Zibet und Castoreum. Als synthetische bzw. halbsynthetische Parfümöle kommen Ambroxan, Eugenol, Isoeugenol, Citronellal, Hydroxycitronellal, Geraniol, Citronellol, Geranylacetat, Citral, lonon und Methylionon in Betracht.
Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

Sowohl Wellmittel als auch Fixiermittel können als Creme, Gel oder Flüssigkeit formuliert sein. Weiterhin ist es möglich, die Mittel in Form von Schaumaerosolen zu konfektionieren, die mit einem verflüssigten Gas wie z. B. Propan-Butan-Gemischen, Stickstoff, Kohlendioxid, Luft, Distickstoffoxid, Dimethylether, Fluorchlorkohlenwasserstofftreibmitteln oder Gemischen davon in Aerosolbehältem mit Schaumventil abgefüllt werden. Die Well- und Fixiermittel können dabei mit allen, dem Fachmann bekannten, üblichen Vorbehandlungsmitteln, Zwischenspülungen und/oder Nachbehandlungsmitteln (zur Verbesserung von Avivage und Haltbarkeit der Frisur) kombiniert werden.

### Beispiele

Die folgenden Beispiele illustrieren die Herstellung von Fixierlösungen auf Basis von Esterquats als Emulgatoren. Zur Herstellung der Zubereitungen wird Wasser auf 75°C erhitzt, die Esterquats sowie gegebenenfalls die weiteren Emulgatoren eingerührt und homogenisiert. Anschließend läßt man die Mischung bis auf 40°C abkühlen und rührt dann die übrigen Inhaltsstoffe ein. Die Zusammensetzung der Fixiermittel ist in Tabelle 1 wiedergegeben.

**Tabelle 1**

| **Fixiermittel (Mengenangaben als Gew.-%)** | | | |
|---|---|---|---|
| **Zusammensetzung (INCI-Bezeichnung)** | **1** | **2** | **3** |
| Dipalmitoylethyl Hydroxyethylmonium Methosulfate, (and) Cetearyl Alcohol (and) Ceteareth-20 | 3,0 | 4,0 | 4,0 |
| Coco Glucosides | - | 1,0 | - |
| Lauridominium Hydroypropyl Hydrolyzed Wheat Protein | - | - | 1,2 |
| Etidronic Acid | 0,3 | 0,3 | 0,3 |
| Wasserstoffperoxid (35 Gew.-%ig) | 7,5 | 7,5 | 7,5 |
| Wasser | ad 100 | | |
| ***pH-Wert*** | 2,5 | 2,7 | 2,8 |
| ***Brookfield-Viskosität (23°C, Sp. TC, 10 Upm)[mPas]*** | 4.900 | 3.600 | 6.150 |

## Patentansprüche

1. Verfahren zur dauerhaften Verformung von Keratinfasern, bei welchem man die Faser vor und/oder nach einer mechanischen Verformung mit einer wäßrigen Zubereitung einer keratinreduzierenden Substanz behandelt, nach einer Einwirkungszeit mit einer ersten Spülung spült, dann mit einer wäßrigen Zubereitung eines Oxidationsmittels fixiert und ebenfalls nach einer Einwirkungszeit spült, **dadurch gekennzeichnet, dass** die wäßrige Zubereitung der keratinreduzierenden Substanz und/oder des Oxidationsmittels Tenside vom Esterquat-Typ mit der Maßgabe enthält, dass nicht gleichzeitig Alkylamidoamine und Aminogruppen aufweisende Polycarbonsäuren im molaren Verhältnis von Alkylamidoainmolekülen zu freien Säuregruppen zwischen 0,9 und 1,1 zugegen sind.

2. Verfahren nach Anspruch 1, **dadurch gekennzeichnet, dass** man Esterquats der Formel **(I)** ein-setzt, 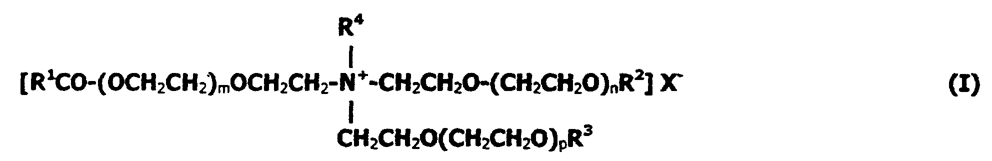 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht

3. Verfahren nach Anspruch 1, **dadurch gekennzeichnet, dass** man Esterquats der Formel **(II)** einsetzt, 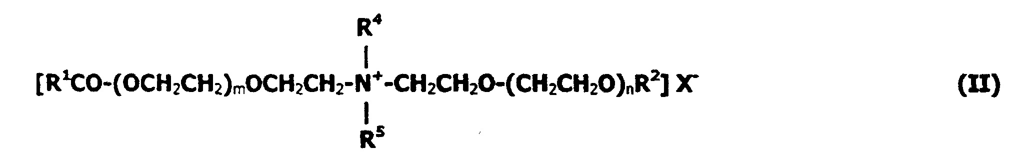 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht

4. Verfahren nach Anspruch 1, **dadurch gekennzeichnet, dass** man Esterquats der Formel **(III)** einsetzt, 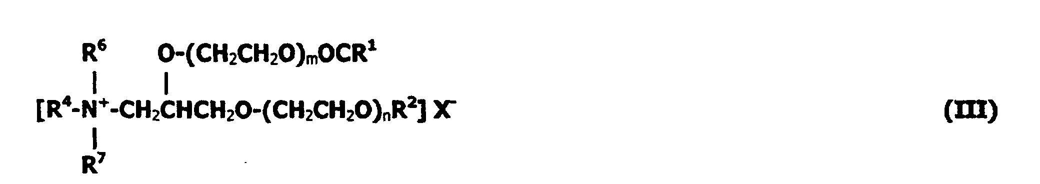 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Verfahren nach den Ansprüchen 1 bis 4, **dadurch gekennzeichnet, dass** man die Esterquats in Mengen von jeweils 0,1 bis 20 Gew.-% - bezogen auf die keratinreduzierende Substanz bzw. das Oxidationsmittel - einsetzt.

6. Verfahren nach den Ansprüchen 1 bis 5, **dadurch gekennzeichnet, dass** man als weitere Tenside Alkyl- und Alkenyloligoglykoside der Formel **(IV)** einsetzt,
**R**^{**8**}**O-[G]**_{**p**} (IV)
in der R⁸ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

7. Verfahren nach den Ansprüchen 1 bis 6, **dadurch gekennzeichnet, dass** man als weitere Tenside Fettsäure-N-alkylpolyhydroxyalkylamide der Formel **(V)** einsetzt, 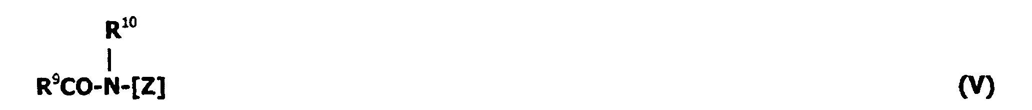 in der R⁹CO für einen aliphatischen Acylrest mit 6 bis 22 Kohlenstoffatomen, R¹⁰ einen Alkyloder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

8. Verfahren nach den Ansprüchen 1 bis 7, **dadurch gekennzeichnet, dass** man als weitere Tenside gegebenenfalls kationisch oder anionisch modifizierte Proteinhydrolysate einsetzt.

9. Verfahren nach den Ansprüchen 1 bis 8, **dadurch gekennzeichnet, dass** man die weiteren Tenside in Mengen von jeweils 0,1 bis 10 Gew.-% - bezogen auf die keratinreduzierende Substanz bzw. das Oxidationsmittel - einsetzt.

## Claims

1. A process for permanently deforming keratin fibres in which the fibres are treated with an aqueous preparation of a keratin-reducing substance before and/or after mechanical deformation, rinsed with a first rinse after a contact time, then fixed with an aqueous preparation of an oxidizing agent and again rinsed after a contact time, **characterized in that** the aqueous preparation of the keratin-reducing substance and/or oxidizing agent contains surfactants of the esterquat type, with the proviso that alkyl amido amines and aminofunctional polycarboxylic acids are not simultaneously present in a molar ratio of alkyl amidoamine molecules to free acid groups of 0.9 to 1.1:1.

2. A process as claimed in claim 1, **characterized in that** esterquats corresponding to formula **(I):** 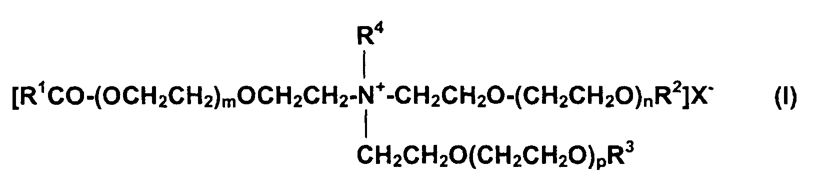 in which R¹CO is an acyl group containing 6 to 22 carbon atoms, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate, are used.

3. A process as claimed in claim 1, **characterized in that** esterquats corresponding to formula **(II):** 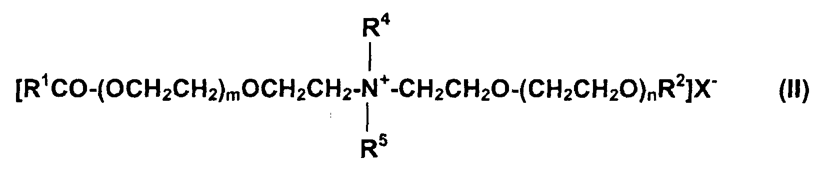 in which R¹CO is an acyl group containing 6 to 22 carbon atoms, R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulfate or alkyl phosphate,
are used.

4. A process as claimed in claim 1, **characterized in that** esterquats corresponding to formula (III): 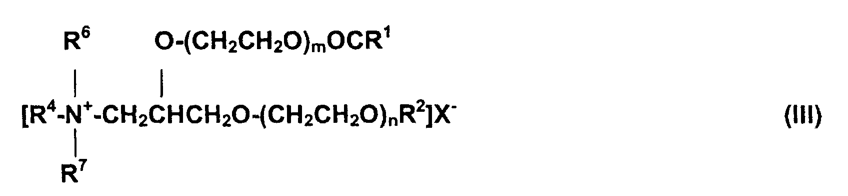 in which R¹CO is an acyl group containing 6 to 22 carbon atoms, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulfate or alkyl phosphate,
are used.

5. A process as claimed in claims 1 to 4, **characterized in that** the esterquats are used in quantities of 0.1 to 20% by weight, based on the keratin-reducing substance or the oxidizing agent.

6. A process as claimed in claims 1 to 5, **characterized in that** alkyl and alkenyl oligoglycosides corresponding to formula **(IV):**
**R**^{**8**}**O-[G]**_{**p**} (IV)
in which R⁸ is an alkyl and/or alkenyl group containing 4 to 22 carbon atoms, G is a sugar unit containing 5 or 6 carbon atoms and p is a number of 1 to 10,
are used as additional surfactants.

7. A process as claimed in claims 1 to 6, **characterized in that** fatty acid-N-alkyl polyhydroxyalkyl amides corresponding to formula (V): 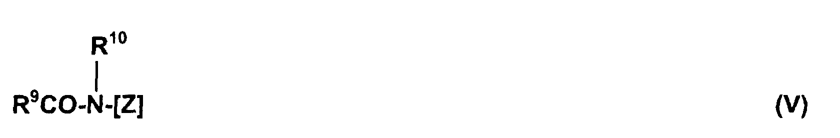 in which R⁹CO is an aliphatic acyl group containing 6 to 22 carbon atoms, R¹⁰ is an alkyl or hydroxyalkyl group containing 1 to 4 carbon atoms and [Z] is a linear or branched polyhydroxyalkyl group containing 3 to 12 carbon atoms and 3 to 10 hydroxyl groups,
are used as additional surfactants.

8. A process as claimed in claims 1 to 7, **characterized in that** optionally cationically or anionically modified protein hydrolyzates are used as additional surfactants.

9. A process as claimed in claims 1 to 8, **characterized in that** the additional surfactants are each used in quantities of 0.1 to 10% by weight, based on the keratin-reducing substance or oxidizing agents.

## Revendications

1. Procédé de mise en forme durable de fibres de kératine, selon lequel avant et/ou après une mise en forme mécanique on traite les fibres avec une préparation aqueuse d'une substance réduisant la kératine, après un temps d'action on les rince avec un premier rinçage, puis on les fixe avec une préparation aqueuse d'un oxydant et on les rince également après un temps d'action,
**caractérisé en ce que**
la préparation aqueuse de la substance réduisant la kératine et/ou de l'oxydant contient des agents tensioactifs de type esters d'ammonium quaternaire, étant précisé qu'elle ne renferme pas simultanément des alkylamidoamines et des acides polycarboxyliques présentant des groupes amino dans un rapport molaire des molécules d'alkylamido amine aux groupes acides libres compris entre 0,9 et 1,1.

2. Procédé selon la revendication 1,
**caractérisé en ce qu'**
on utilise des esters d'ammonium quaternaire de formule (I) 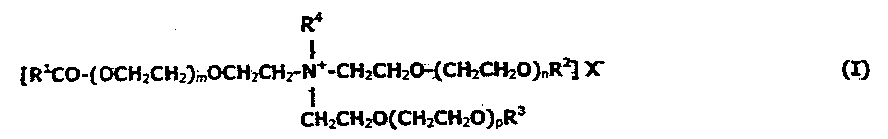 dans laquelle R¹CO représente un radical acyle comportant de 6 à 22 atomes de carbone, R² et R³ représentent indépendamment l'un de l'autre un hydrogène ou R¹CO, R⁴ représente un radical alkyle comportant de 1 à 4 atomes de carbone ou un groupe (CH₂CH₂O)_{q}H, m, n et p valent au total 0 ou des nombres allant de 1 à 12, q représente des nombres allant de 1 à 12 et X représente un halogénure, un sulphate d'alkyle ou un phosphate d'alkyle.

3. Procédé selon la revendication 1,
**caractérisé en ce qu'**
on utilise des esters d'ammonium quaternaire de formule (II), 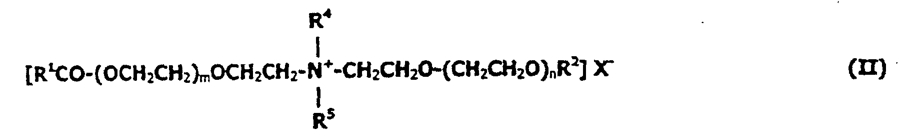 dans laquelle R¹CO représente un radical acyle comportant de 6 à 22 atomes de carbone, R² représente un hydrogène ou R¹CO, R⁴ et R⁵ représentent indépendamment l'un de l'autre des radicaux alkyle comportant de 1 à 4 atomes de carbone, n et m valent au total 0 ou des nombres allant de 1 à 12 et X représente un halogénure, un sulfate d'alkyle ou un phosphate d'alkyle.

4. Procédé selon la revendication 1,
**caractérisé en ce qu'**
on utilise des esters d'ammonium quaternaire de formule (III) 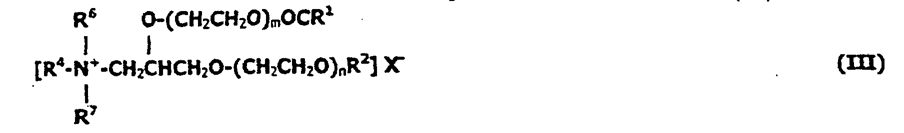 dans laquelle R¹CO représente un radical acyle comportant de 6 à 22 atomes de carbone, R² représente un hydrogène ou R¹CO, R⁴, R⁶ et R⁷ représentent indépendamment les uns des autres des radicaux alkyle comportant de 1 à 4 atomes de carbone, m et n valent au total 0 ou des nombres allant de 1 à 12 et X représente un halogénure, un sulfate d'alkyle ou un phosphate d'alkyle.

5. Procédé selon les revendications 1 à 4,
**caractérisé en ce qu'**
on utilise les esters d'ammonium quaternaire en des quantités respectives de 0,1 à 20 % en poids - par rapport à la substance réduisant la kératine, ou à l'oxydant.

6. Procédé selon les revendications 1 à 5,
**caractérisé en ce que**
comme autres agents tensioactifs on utilise des alkyl- et alcényl-oligoglycosides de formule (IV)
R⁸O-[G]ₚ (IV)
dans laquelle R⁸ représente un radical alkyle et/ou alcényle comportant de 4 à 22 atomes de carbone, G représente un radical saccharique comportant 5 ou 6 atomes de carbone et p représente des nombres allant de 1 à 10.

7. Procédé selon les revendications 1 à 6,
**caractérisé en ce que**
comme autres agents tensioactifs on utilise des N-alkylpolyhydroxyalkylamides d'acides gras de formule (V) 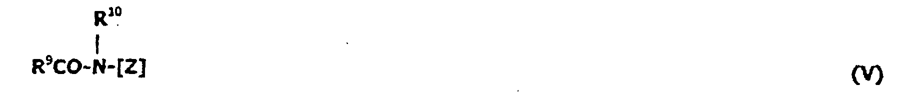 dans laquelle R⁹CO représente un radical acyle aliphatique comportant de 6 à 22 atomes de carbone, R¹⁰ représente un radical alkyle ou hydroxyalkyle comportant de 1 à 4 atomes de carbone et [Z] représente un radical polyhydroxyalkyle linéaire ou ramifié comportant de 3 à 12 atomes de carbone et de 3 à 10 groupes hydroxyle.

8. Procédé selon les revendications 1 à 7,
**caractérisé en ce que**
comme autres agents tensioactifs on utilise des hydrolysats de protéines éventuellement à modification cationique ou anionique.

9. Procédé selon les revendications 1 à 8,
**caractérisé en ce qu'**
on utilise les autres agents tensioactifs en quantités respectives de 0,1 à 10 % en poids - par rapport à la substance réduisant la kératine, ou à l'oxydant.
